(19) Europäisches Patentamt
European Patent Office
Office européen des brevets

(11) Veröffentlichungsnummer : **0 174 542**
**B1**

(12) # EUROPÄISCHE PATENTSCHRIFT

(45) Veröffentlichungstag der Patentschrift : **28.06.89**

(21) Anmeldenummer : **85110655.9**

(22) Anmeldetag : **24.08.85**

(51) Int. Cl.⁴ : **C 07 C 93/04**, C 07 F 15/00, C 07 D319/06, A 61 K 31/555

(54) **Cis-Platin-Komplexe mit einem Pentaerythritderivat als Liganden, Verfahren zu ihrer Herstellung und diese Verbindungen enthaltendes pharmazeutisches Mittel.**

(30) Priorität : **03.09.84 DE 3432320**

(43) Veröffentlichungstag der Anmeldung : **19.03.86 Patentblatt 86/12**

(45) Bekanntmachung des Hinweises auf die Patenterteilung : **28.06.89 Patentblatt 89/26**

(84) Benannte Vertragsstaaten : **AT BE CH DE FR GB IT LI LU NL SE**

(56) Entgegenhaltungen :
**DE–A– 2 927 056**
**GB–A– 2 128 615**

(73) Patentinhaber : **BEHRINGWERKE Aktiengesellschaft**
**Postfach 1140**
**D-3550 Marburg 1 (DE)**

(72) Erfinder : **Kolar, Cenek, Dr.**
**Deutschhausstrasse 20**
**D-3550 Marburg 1 (DE)**
Erfinder : **Kraemer, Hans Peter, Dr.**
**Birkenweg 16**
**D-3550 Marburg (DE)**
Erfinder : **Dehmel, Konrad**
**Blumengarten 9**
**D-3550 Marburg-Michelbach (DE)**

(74) Vertreter : **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 174 542 B1**

Jouve, 18, rue St-Denis, 75001 Paris, France

## Beschreibung

Die Erfindung betrifft neue Platinverbindungen, ein Verfahren zu ihrer Herstellung und ein pharmazeutisches Mittel, in dem diese neuen Verbindungen enthalten sind. Es handelt sich um Platin-(II)-diamin-Komplexe der Formeln I und II

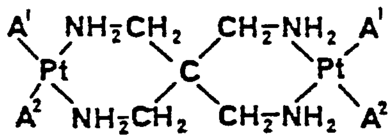

Formel I

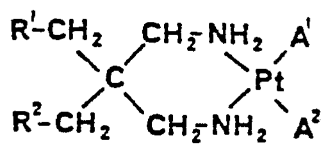

Formel II

worin

$R^1$ und $R^2$ unabhängig voneinander für $H—(CH_2)_a—(0—(CH_2)_b)_c—O$ mit $a = 0$ bis 4, $b = 1$ bis 4 und $c = 1$ bis 7, eine Alkyloxy- oder Arylalkyloxygruppe mit 1 bis 20 C-Atomen, eine Alkan- oder Arylalkansulfonyloxygruppe mit 1 bis 7 C-Atomen oder einen Tetrahydropyranyloxyrest stehen oder

$R^1$ und $R^2$ zusammen ein etherartig gebundenes Sauerstoffatom oder einen Acetal- oder Ketal-Rest

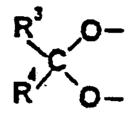

mit

$R^3$ und $R^4$ unabhängig voneinander ein Wasserstoffatom, eine Alkylgruppe mit 1 bis 20 C-Atomen oder eine Phenylgruppe,

$A^1$ und $A^2$ gleich sind und die Hydroxygruppe, Chlorid, Bromid, Jodid, Nitrat, Acetat, Trifluoracetat, Trifluorsulfonat, oder Perchlorat oder

$A^1$ Sulfat oder Carbonat und $A^2$ $H_2O$ oder

$A^1$ und $A^2$ zusammen das Dianion einer organischen Säure, wie Oxal-, Malon-, Hydroxymalon-, Ethylmalon-, Bernstein-, Malein-, Aconit-, 3,6,9-Trioxaundecandi-, 1,1- oder 1,2-Cyclobutandicarbon-, Phthal-, 3- oder 4-Carboxyphthal- oder 3,4-Dicarboxyphthalsäure oder

$A^1$ und $A^2$ zusammen eine wiederkehrende anionische Einheit einer polymeren Verbindung wie Dextran-, Chondroitin-, oder Galactansulfat, Polyglutaminsäure oder Polyitaconsäure darstellen.

Es ist bekannt, daß cis-Platin-Komplexe der allgemeinen Formel cis-$L_2PtX_2$, wobei L ein neutraler Ligand wie $NH_3$ oder organisches Amin und X ein anionischer Ligand wie Chlorid oder ein Anion einer organischen Säure ist, Antitumorwirkung besitzen (Cisplatin — Current Status and New Developments; Eds. A.W. Prestayko, S.T. Crooke, S.K. Carter, Academic Press, 1980). Cis-platin-II-diamindichlorid ist als Arzneimittel eingeführt.

In DE 3 337 333 Al und NL-OS 7 904 740 sind cis-Platin-Komplexe beschrieben, die als Liganden Alkyl-, Aryl- oder Arylalkylpropyl-1,3-diamin-Derivate besitzen.

IN EPA 0 098 135 sind ebenfalls Propyl-1,3-diamino-Liganden beschrieben, die Alkyl- oder Hydroxyalkyl-Substituenten tragen.

Die Nieren- und Knochenmarktoxizität der Alkyldiaminplatin-Komplexe sowie ihre geringe Löslichkeit sind von Nachteil.

Die vorliegende Erfindung hatte sich zur Aufgabe gestellt, neue cis-Platin-Komplexe herzustellen, in denen die Pentaerythrit-Struktur als zweizähniger Diaminoligand fungiert und eine therapeutisch vertretbare anionische Verbindung als Ligand vorliegt, und die weniger nieren- und knochenmarktoxisch sind als die in der Klinik eingeführte cis-Platin-II-diamindichlorid-Verbindung.

Gelöst wurde diese Aufgabe durch die Herstellung von Verbindungen mit den voranstehenden Formeln I und II und den zu diesen Formeln angegebenen Definitionen sowie durch deren Prüfung auf zytostatische Wirksamkeit.

Es wurde überraschenderweise gefunden, daß die Pentaerythrit-Derivat-cis-platin-Komplexe der allgemeinen Formeln I und II zytostatisch wirksam sind und wesentlich günstigere biologische Eingenschaften wie niedrige Nieren- und Knochenmarktoxizität sowie physikalische Eigenschaften wie bessere Löslichkeit in wäßrigem Medium aufweisen als die Stammverbindung cis-$(NH_3)_2PtCl_2$.

Bevorzugt werden im Rahmen der Erfindung Verbindungen der allgemeinen Formeln I, III, IV und V

(Siehe Formeln Seite 3 f.)

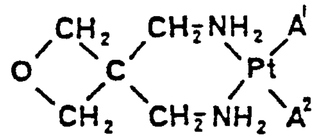

Formel III

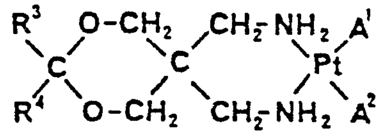

Formel IV

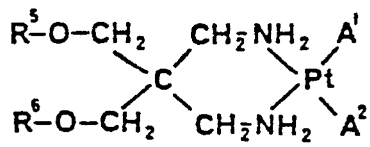

Formel V

worin

$R^3$ und $R^4$ unabhängig voneinander H, $-(CH_2)_n-CH_3$ mit n = 0, 9 oder 17 oder Phenyl,

$R^5$ und $R^6$ unabhängig voneinander $CH_3-(OCH_2CH_2)_c-$ mit c = 3 oder 6, $-(CH_2)_nCH_3$ mit n = 0, 9 oder 17, eine Tetrahydropyranyl-, Methansulfonyl- oder para-Toluolsulfonylgruppe,

$A^1$ und $A^2$ gleich sind und Chlorid, Jodid, Nitrat, Hydroxy, Acetat, Trifluoracetat, Trifluorsulfonat, p-Toluolsulfonat oder Perchlorat oder

$A^1$ Sulfat oder Carbonat und $A^2$ $H_2O$,

$A^1$ und $A^2$ zusammen das Dianion Malon-, Malein-, 1,1- oder 1,2-Cyclobutandicarbon-, 3- oder 4-Carboxyphthal- oder 3,6,9-Trioxaundecandisäure oder

$A^1$ und $A^2$ zusammen eine wiederkehrende anionische Einheit von Polyglutaminsäure oder Chondroitinsulfat darstellen.

Das erfindungsgemäße Verfahren zur Herstellung der Verbindungen der Formeln I und II ist dadurch gekennzeichnet, daß man

a) in an sich bekannter Weise Pentaerythrit zu Neopentyltetramin oder zu einer Neopentyldiazid-Verbindung der allgemeinen Formel VI

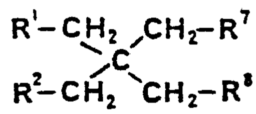

Formel VI

worin

$R^1$ und $R^2$ unabhängig voneinander für OH, $H-(CH_2)_a-(0-(CH_2)_b)_c-O$ mit a = 0 bis 4, b = 1 bis 4, c = 1 bis 7, eine Alkyloxy- oder Arylalkyloxygruppe mit 1 bis 20 C-Atomen, Alkan- oder Arylalkansulfonyloxygruppe mit 1 bis 7 C-Atomen oder einen Tetrahydropyranyloxyrest stehen oder

$R^1$ und $R^2$ zusammen ein etherartig gebundenes Sauerstoffatom oder einen Rest

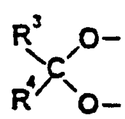

mit

$R^3$ und $R^4$ unabhängig voneinander ein Wasserstoffatom, eine Alkylgruppe mit 1 bis 20 C-Atomen oder eine Phenylgruppe und

$R^7$ und $R^8$ Azidogruppen sind, umsetzt und die erhaltene Diazido-Verbindung in Gegenwart von Palladium/Kohle und eines organischen Lösungsmittels wie Methanol, Essigsäureethylester oder Dioxan hydriert oder mit einem Reduktionsmittel wie Natriumborhydrid reduziert,

wobei eine Diamino-Verbindung der Formel VI gebildet wird, in der $R^1$, $R^2$, $R^3$ und $R^4$ unverändert und $R^7$ und $R^8$ Aminogruppen sind ;

b) das Produkt von Stufe a) in an sich bekannter Weise mit $K_2PtX_4$, worin X ein Chlorid- oder Jodid-Ion darstellt, in Gegenwart eines Lösungsmittels wie Wasser, DMF oder DMSO oder deren Mischungen mit THF, Dioxan, Methanol oder Ethylenglycoldimethylether bei einer Temperatur von 0 °C bis 80 °C, bevorzugt 10 °C bis 40 °C, zu Reaktionsprodukten der allgemeinen Formeln I, III, IV und V umsetzt, worin

die Reste $R^3$, $R^4$, $R^5$ und $R^6$ die bereits angeführte Bedeutung besitzen und $A^1$ und $A^2$ Chlorid oder Jodid sind ;

c) das Produkt von Stufe b) in an sich bekannter Weise mit einer anorganischen oder organischen Silber-I-Verbindung wie Silberoxid, -nitrat, -sulfat, -carbonat, -perchlorat, -acetat, trifluoracetat, -trifluorsulfonat oder -para-toluolsulfonat in Gegenwart von Wasser oder einem organischen Lösungsmittel, wie DMF, DMSO, THF, Dioxan oder Methanol oder deren wäßrigen Mischungen zu Verbindungen der allgemeinen Formeln I, III, IV und V umsetzt, worin $A^1$ und $A^2$ Hydroxygruppen oder ein Anion der eingesetzten Silberverbindung darstellen ;

d) in dem Produkt von Stufe b) in an sich bekannter Weise die anionischen Liganden $A^1$ und $A^2$, bevorzugt Nitrat oder Perchlorat, gegen eine anionische Verbindung, die sich von einem Alkalisalz einer Carbon- oder Oligocarbonsäure ableitet, austauscht. Solche anionischen Verbindungen sind Dicarbonsäuren wie Oxal-, Malon-, Hydroxymalon-, Bernstein-, Maleinsäure, 1,1- oder 1,2-Cyclobutandicarbonsäure, Phthalsäuren, 3,6,9-Trioxaundecandisäure, Tri- oder Oligocarbonsäuren wie Aconitsäure, 3- oder 4-Carboxyphthalsäure, 3,4-Dicarboxyphthalsäure, polymere Verbindungen wie Polyglutaminsäure, Polyitaconsäure, Dextrasulfat, Chondroitinsulfat oder Galactansulfat.

Gegenstand der Erfindung sind auch Arzneimittel, die eine oder mehrere der Verbindungen der Formeln I und II als Wirkstoff enthalten.

Neben den üblichen pharmazeutischen Konfektionierungs- und/oder Verdünnungsmitteln können diese Arzneimittel neben den Verbindungen der Formeln I und II zur Unterstützung der Therapie gegebenenfalls auch noch weitere Wirkstoffe enthalten, sofern diese mit den erfindungsgemäßen Verbindungen der Formeln I und II zusammen keine unerwünschten Nebenwirkungen zeigen. Die Dosierungs- und Anwendungsweise entspricht im wesentlichen der für cis-$(NH_3)_2PtCl_2$ bekannten, wobei aber aufgrund der größeren therapeutischen Breite (geringere Toxizität) der erfindungsgemäßen Verbindungen auch höhere Dosierungen und/oder eine häufigere Verabreichung in Frage kommen.

Ermittlung der zytotoxischen Aktivität

Die Bestimmung der zytostatischen Wirksamkeit der hier beschriebenen Verbindungen erfolgte an L1210 Leukämiezellen der Maus. Im einzelnen wurden folgende Testsysteme verwendet :

a) Proliferationsassay

Bei dieser Methode wird in vitro nach Inkubation der Zellen mit unterschiedlichen Konzentrationen der Testsubstanz ermittelt, inwieweit die Zellen radioaktiv markierte DNA-Vorläufer (z. B. C14 markiertes Thymidin) einbauen können. Unbehandelte L1210 Zellen werden den gleichen Testbedingungen unterworfen und dienen als Kontrolle. Im folgenden sei die Methode kurz beschrieben :

L1210 Zellen in expotentieller Wachstumsphase ($5 \times 10^3$/ml in RPMI 1640) werden in einer Mikrotitrationsplatte 72 Stunden mit unterschiedlichen Konzentrationen der Testsubstanz inkubiert (37 °C, 5 Vol-% $CO_2$, 95 % relative Luftfeuchte). Kontrollen bestehen aus Zellen, die lediglich mit frischem Medium inkubiert werden. Alle Bestimmungen werden als 4-fach Bestimmungen durchgeführt. Nach 65 Stunden werden 50 µl C14 Thymidin (1,5 µc/ml) zugegeben, um die DNA der Zelle radioaktiv zu markieren. Nach 7 Stunden Inkubation werden die Zellen abgesaugt, die DNA mit 5 %iger Trichloressigsäure gefällt und nacheinander mit Wasser oder Methanol gewaschen.

Nach Trocknung bei 50 °C wird die in die DNA eingebaute Radioaktivität nach Zugabe von 5 ml Szintilationsflüssigkeit ermittelt.

Die Ergebnisse werden angegeben als Verhältnis des Szintilationsindex nach Inkubation mit der Testsubstanz in Prozent der unbehandelten Kontrolle. Aus den so erhaltenen Meßwerten wird die Dosiswirkungs kurve ermittelt und grafisch die $IC_{50}$, d. h. die Konzentration, die unter Testbedingungen den Einbau von radioaktivem Thymidin um 50 % gegenüber der Kontrolle erniedrigt, ermittelt. Die $IC_{50}$ Werte der hier beschriebenen Verbindungen im Vergleich zu Cisplatin (DDP) wurden in der Tabelle 1 zusammengefaßt.

b) Koloniebildung von L1210 Leukämiezellen in soft agar

Diese Methode dient zum Nachweis eines Einflusses der Testsubstanzen auf das Wachstumsverhalten der Zellen über mehrere Generationen (bei einer Zellzykluszeit von 10-12 Stunden werden in der Testzeit von 7 Tagen etwa 14 aufeinanderfolgende Generationen beobachtet). Zytostatisch wirksame Substanzen bewirken in diesem Test eine Reduktion der zu beobachtenden Koloniezahl gegenüber einer unbehandelten Kontrolle. Im einzelnen wird der Test wie folgt durchgeführt :

500 Leukämiezellen pro Platte werden mit unterschiedlichen Konzentrationen von Testsubstanz 1 Stunde bei 37 °C inkubiert. Anschließend werden die Zellen zweimal mit McCoy5A-Medium gewaschen und schließlich in Petrischalen nach Zusatz von 0.3 % Agar ausgegossen. Kontrollen werden lediglich mit frischen Medium inkubiert. Anstelle der einstündigen Inkubation werden in manchen Fällen unterschiedliche Konzentrationen und Testsubstanz der oberen Agarschicht zugemischt, um so eine kontinuierliche Exposition der Zellen über die gesamte Inkubationszeit zu erreichen. Nach Erstarren des Agars werden die Platten im Brutschrank 7 Tage bei 37 °C inkubiert (5 Vol-% $CO_2$, 95 % relative Luftfeuchtigkeit). Anschließend wird die Anzahl der entstandenen Kolonien mit einem. Durchmesser von mehr als 60 μ gezählt. Die Ergebnisse werden angegeben als Koloniezahl in behandelten Agarplatten in Prozent der unbehandelten Kontrolle. Aus der so erhaltenen Dosiswirkungskurve wird die $IC_{50}$ als Maß für die Wirksamkeit der Substanz ermittelt. Die Ergebnisse für die hier beschriebenen Verbindungen im Vergleich zu Cisplatin sind in der Tabelle 1 zusammengefaßt.

c) Ermittlung der akuten Toxizität

Zur Ermittlung der akuten Toxizität werden NMRI-Mäusen am Tag 0 unterschiedliche Dosen der Testsubstanz, gelöst in 0.5 ml physiologischer Kochsalzlösung, intravenös injiziert. Kontrollgruppen erhalten lediglich 0,5 ml physiologischer Kochsalzlösung. Pro Konzentration der Testsubstanz werden 5 Mäuse verwendet. Am Tag 14 wird die Zahl der überlebenden Mäuse ermittelt und daraus nach der Litchfield Wilcoxon Methode die LD5, LD50 und LD95 ermittelt. Die Toxizität der hier beschriebenen Verbindungen im Vergleich zu Cisplatin ist in Tabelle 1 zusammengefaßt.

Tabelle 1

Ermittlung der zytotoxischen Aktivität

| Verbindung | a | b | c |
|---|---|---|---|
| 44 | - | 0,34 | - |
| 45 | - | 0,23 | - |
| 46 | - | 0,26 | - |
| 57 | 1,9 | 0,25 | 3,4 |
| 50 | - | 0,18 | - |

Beispiele

Die Struktur der folgenden Verbindungen wurde mittels Elementaranalyse, $^1H$-, bzw. $^{13}C$-NMR-sowie IR-Spektroskopie ermittelt.

Der Verlauf der Reaktionen sowie die resultierenden Produkte wurden dünnschichtchromatographisch und mit der HPLC-Technik untersucht.

Die folgenden Beispiele erläutern die Erfindung näher, ohne sie darauf zu beschränken.

Beispiel 1

2,2-Bis-(hydroxymethyl)-1,3-diazidopropan (Verbindung 1)

a) 1 Mol Pentaerythrit wurde in 600 ml trockenem Pyridin gelöst. Unter Kühlen wurden der gerührten Lösung 2 Mol para-Toluolsulfonsäurechlorid zugegeben. Danach wurde die Reaktionsmischung 10 Stunden bei Raumtemperatur gerührt. Nach Einengen der abfiltrierten Lösung im Vakuum wurde der resultierende Sirup in Chloroform gelöst und zweimal mit Wasser ausgeschüttelt. Die organische Phase wurde getrocknet und im Vakuum eingeengt. Das Produkt wurde in einer Mischung aus 500 ml Ethanol und 400 ml Wasser gelöst und mit 3 Mol Natriumazid und 3 Mol Ammoniumchlorid versetzt. Nach 24 h Kochen unter Rückfluß wurde die Reaktionsmischung im Vakuum zum Sirup eingeengt und mit Essigsäureethylester/Wasser gewaschen. Die organische Phase wurde getrocknet und im Vakuum zum Sirup eingeengt. Das Produkt wurde noch säulenchromatographisch gereinigt (Elutionsmittel : Chloroform/Aceton 4 : 1).

Ausbeute : 62 %

b) 1 Mol Pentaerythrit wurde mit 1 Mol Benzaldehyddimethylacetal, gelöst in 500 ml trockenem DMF, und katalytischen Menge von para-Toluolsulfonsäure versetzt. Nach 24 h Rühren bei 60 °C wurde die abgekühlte Reaktionsmischung in 1 000 ml Eiswasser gegossen und das Reaktionsprodukt in Diethylether aufgenommen. Die organische Phase wurde getrocknet und im Vakuum eingeengt. Das resultierende Produkt wurde in 600 ml trockenem Pyridin gelöst, und unter Kühlen wurden 2 Mol Methansulfonsäurechlorid gelöst in 300 ml trockenem Chloroform zugetropft. Danach wurde die Reaktionsmischung 10 Stunden bei Raumtemperatur gerührt. Die Reaktionsmischung wurde im Vakuum zum Sirup eingeengt

und mit Chloroform/Wasser gewaschen. Die organische Phase wurde getrocknet und im Vakuum zum Sirup eingeengt. Das resultierende Produkt wurde in einer Mischung aus 500 ml Ethanol und 300 ml Wasser gelöst, mit 3 Mol Natriumazid und 2 Mol Ammoniumchlorid versetzt und 24 h unter Rückfluß gekocht. Die Reaktionsmischung wurde zum Sirup eingeengt. Das Produkt wurde in Chloroform suspendiert und mit Wasser dreimal gewaschen. Die organische Phase wurde getrocknet und im Vakuum zum Sirup eingeengt. Die Abspaltung der Benzylidengruppe aus dem Reaktionsprodukt wurde in einer Mischung aus Essigsäure/Schwefelsäure 100 : 1 bei 60 °C durchgeführt. Nach der Umsetzung wurde die Schwefelsäure mit Natriumacetat neutralisiert, und es wurde im Vakuum zum Sirup eingeengt. Das Produkt wurde noch säulenchromatographisch gereinigt.

Ausbeute : 73 %

IR ($cm^{-1}$) : 3 400, 3 040-2 900, 2 100

Beispiel 2

2,2-Bis-(methoxymethyl)-1,3-diazidopropan (Verbindung 2)

0,5 Mol Verbindung 1 wurden in 600 ml trockenem Dioxan gelöst. Unter Feuchtigkeitsausschluß wurden 1,2 Mol Kalium tert.-butylat und 1,1 Mol Methyljodid bei 5 °C zugegeben. Nach 15 Minuten Rühren bei Raumtemperatur wurde die Reaktionsmischung im Vakuum eingeengt. Der in 100 ml Ether aufgenommene Rückstand wurde zweimal mit 5 %-iger (w : v) wäßriger NaCl-Lösung gewaschen. Die organische Phase wurde mit Natriumsulfat getrocknet und im Vakuum zum Sirup eingeengt. Das resultierende Produkt wurde noch säulenchromatographisch gereinigt (Elutionsmittel : Chloroform/Essigsäureethylester 7 : 1).

Ausbeute : 93 %

Beispiel 3

2,2-Bis-(octadecyloxymethyl)-1,3-diazidopropan (Verbindung 3)

0,5 Mol Verbindung 1 wurden in 500 ml trockenem Toluol gelöst. Nach Zugabe von 1,2 Mol Kalium tert.-butylat und 1,05 Mol Methansulfonsäureoctadecylester wurde die Suspension auf 70 °C erwärmt und 40 Minuten unter Stickstoffatmosphere gerührt. Nach Abkühlen wurde die Reaktionsmischung mit Toluol versetzt, und die Salze wurden mit Wasser ausgewaschen. Nach Einengen der organischen Phase wurde das Rohprodukt chromatographisch gereinigt (Elutionsmittel : Chloroform/Essigester 10 : 1).

Ausbeute : 82 %

Analog wurde folgende Verbindung synthetisiert 2,2-Bis-(decyloxymethyl)-1,3-diazidopropan (Verbindung 4)

Analog wurden weiterhin folgende Verbindungen synthetisiert, jedoch wurden pro 0,5 Mol Verbindung 1 0,6 Mol Kalium-tert.-butylat und 0,55 Mol Methansulfonsäureoctadecylester oder -decylester in die Reaktion eingesetzt.

2-Hydroxymethyl-2-octadecyloxymethyl-1,3-diazidopropan (Verbindung 5)

2-Decycloxymethyl-2-hydroxymethyl-1,3-diazidopropan (Verbindung 6)

Wie in Beispiel 2 beschrieben, wurden die Verbindungen 5 und 6 zu Methoxy-Derivaten umgesetzt.

2-Methoxymethyl-2-octadecyloxymethyl-1,3-diazidopropan (Verbindung 7)

2-Decyloxymethyl-2-methoxymethyl-1,3-diazidopropan (Verbindung 8)

Beispiel 4

Wie im Beispiel 1 beschrieben, wurden die folgenden Methan- oder para-Toluolsulfonsäureester-Verbindungen ausgehend von Verbindung 1 hergestellt.

2-Hydroxymethyl-2-para-toluolsulfonyloxymethyl-1,3-diazidopropan (Verbindung 9)

2,2-Bis-(para-toluolsulfonyloxymethyl)-1,3-diazidopropan (Verbindung 10)

2,2-Bis-(Methansulfonyloxymethyl)-1,3-diazidopropan (Verbindung 11)

Beispiel 5

3,3-Bis-(azidomethyl)-oxetan (Verbindung 12) 0,1 Mol Verbindung 9 wurden in 100 ml trockenem Dioxan gelöst. Unter Feuchtigkeitsausschluß wurden 0,15 Mol Kalium-tert.-butylat zugegeben. Nach 3 h Rühren bei 60 °C wurde die Reaktionsmischung eingeengt. Der in 400 ml Ether aufgenommene Rückstand wurde zweimal mit 5 %-iger (w : v) wäßriger NaCl-Lösung gewaschen. Die organische Phase wurde getrocknet und im Vakuum eingeengt. Das einheitliche Produkt wurde noch säulenchromatographisch gereinigt (Elutionsmittel : Chloroform/Essigsäureethylester 5 : 1).

Ausbeute : 62 %

Beispiel 6

5,5-Bis-(azidomethyl)-1,3-dioxan (Verbindung 13)

Wie in Beispiel 1a) wurde Verbindung 13 ausgehend von Pentaerythritmonomethylenether hergestellt.

Beispiel 7

5,5-Bis-(azidomethyl)-2-phenyl-1,3-dioxan (Verbindung 14)

Die Herstellung von Verbindung 14 wurde bereits als eine Zwischenstufe im Beispiel 1b) beschrieben.

Beispiel 8

5,5-Bis-(azidomethyl)-2,2-bis-(methyl)-1,3-dioxan (Verbindung 15)

1 Mol Verbindung 1 wurde in 500 ml trockenem Aceton gelöst. Nach der Zugabe von 10 ml konzentrierter Schwefelsäure wurde die Reaktionsmischung 2 h bei Raumtemperatur gerührt. Es wurde mit Calciumhydroxyd neutralisiert, abfiltriert und im Vakuum zum Sirup eingeengt. Das Rohprodukt wurde noch säulenchromatographisch gereinigt (Elutionsmittel : Chloroform/Essigsäureethylester 9 : 1).

Ausbeute : 84 %

Beispiel 9

5,5-Bis-(azidomethyl)-2-methyl-2-octadecyl-1,3-dioxan (Verbindung 16)

0,8 Mol Methyl-octadecyl-keton wurden mit 1 Mol Verbindung 1 und 0,1 g p-Toluolsulfonsäure in 250 ml Toluol am Wasserabscheider unter Rückfluß getrocknet, bis sich kein Reaktionswasser mehr bildete. Nach Abkühlen wurde die Reaktionsmischung mit verdünnter Natronlauge neutralisiert und im Vakuum zum Sirup eingeengt. Das Produkt wurde noch säulenchromatographisch gereinigt (Elutionsmittel : Dichlormethan/Essigsäureethylester 6 : 1).

Ausbeute : 60 %

Wie im Beispiel 9 beschrieben, wurde folgende Verbindung ausgehend von Methyl-octadecyl-keton und Verbindung 1 analog hergestellt. 5,5-Bis-(azidomethyl)-2-decyl-2-methyl-1,3-dioxan (Verbindung 17)

Beispiel 10

Die Diazido-Verbindungen 2 bis 17 wurden nach der folgenden allgemeinen Arbeitsvorschrift zu ihren Diamino-Derivaten umgesetzt.

0,1 Mol einer Diazido-Verbindung wurden in 400 ml eines Gemisches aus Methanol/Essigsäureethylester 2 : 1 gelöst. Nach der Zugabe von 15 g Palladium/Kohle (10 %) wurde der Reaktionsansatz unter Rühren 3 h bei Raumtemperatur hydriert. Der Verlauf der Hydrierung wurde dünnschichtchromatographisch (Laufmittel : Chloroform/Methanol/Wasser 4 : 2 : 1 oder 2 : 2 : 1) verfolgt. Dann wurde abfiltriert und im Vakuum zum Sirup eingeengt. Das resultierende Produkt, das in IR-Spektrum keine Azidobande zeigte, wurde ohne weitere Reinigungschritte in die folgende Umsetzung mit Platinsalzen eingesetzt.

Ausbeute : 75 bis 96 %

2,2-Bis-(methoxymethyl)-1,3-diaminopropan (Verbindung 18)
2,2-Bis-(octadecyloxymethyl)-1,3-diaminopropan (Verbindung 19)
2,2-Bis-(decyloxymethyl)-1,3-diaminopropan (Verbindung 20)
2-Hydroxymethyl-2-octadecyloxymethyl-1,3-diaminopropan (Verbindung 21)
2-Decyloxymethyl-2-hydroxymethyl-1,3-diaminopropan (Verbindung 22)
2-Methoxymethyl-2-octadecyloxymethyl-1,3-diaminopropan (Verbindung 23)
2-Decyloxymethyl-2-methoxymethyl-1,3-diaminopropan (Verbindung 24)
2-Hydroxymethyl-2-para-toluolsulfonyloxymethyl-1,3-diamonopropan (Verbindung 25)
2,2-Bis-(para-toluolsulfonyloxymethyl-1,3-diaminopropan (Verbindung 26)
2,2-Bis-(Methansulfonyloxymethyl)-1,3-diaminopropan (Verbindung 27)
3,3-Bis-(aminomethyl)-oxetan (Verbindung 28)
5,5-Bis-(aminomethyl)-1,3-dioxan (Verbindung 29)
5,5-Bis-(aminomethyl)-2-phenyl-1,3-dioxan (Verbindung 30)
5,5-Bis-(aminomethyl)-2,2-bis-(methyl)-1,3-dioxan (Verbindung 31)
5,5-Bis-(aminomethyl)-2-methyl-2-octadecyl-1,3-dioxan (Verbindung 32)
5,5-Bis-(aminomethyl-2-decyl-2-methyl-1,3-dioxan (Verbindung 33)

Beispiel 11

Nach der folgenden allgemeinen Arbeitsvorschrift wurden die Diamino-Verbindungen 18 bis 33 in Platinkomplexe umgesetzt.

60 mMol Diamino-Verbindung wurden in 50 ml destilliertem, entgasten Wasser gelöst. Unter Rühren wurde die Lösung innerhalb 2 Stunden zu 60 mMol Kaliumtetrachloroplatinat, gelöst in 60 ml Wasser, getropft. Der Reaktionsansatz wurde noch 20 Stunden unter Lichtausschluß bei Raumtemperatur gerührt, wobei im Verlauf der Reaktion der Platinkomplex als Niederschlag ausfiel. Das Dünnschichtchromatogramm der Reaktion (Laufmittel : Chloroform/Methanol/Wasser 4 : 4 : 1 ; Sprühreagenzien : Ninhydrin und Zinndichlorid) zeigte, daß die Ninhydrin-aktive Ausgangsverbindung vollständig umgesetzt war. Der Niederschlag wurde bei 0 °C abfiltriert, mit kaltem Wasser gewaschen und gegebenenfalls aus DMF und Wasser umkristallisiert. Das Filtrat wurde im Vakuum auf etwa 15 ml Volumen eingeengt und auf 0 °C abgekühlt. Die ausgefallene Verbindung wurde in gleicher Weise wie die Hauptfraktion aufgearbeitet. Das Produkt wurde noch 3 Tage im Hochvakuum getrocknet.

Ausbeute : 80 % bis 92 %.

2,2-Bis-(methoxymethyl)-1,3-diamonopropan-platin-II-dichlorid (Verbindung 34)
2,2-Bis-(octadecyloxymethyl)-1,3-diaminopropan-platin-II-dichlorid (Verbindung 35)
2,2-Bis-(decyloxymethyl)-1,3-diaminopropan-platin-II-dichlorid (Verbindung 36)
2-Hydroxymethyl-2-octadecyloxymethyl-1,3-diaminopropan-platin-II-dichlorid (Verbindung 37)
2-Decyloxymethyl-2-hydroxymethyl-1,3-diaminopropan-platin-II-dichlorid (Verbindung 38)
2-Methoxymethyl-2-octadecyloxymethyl-1,3-diaminopropan-platin-II-dichlorid (Verbindung 39)
2-Decyloxymethyl-2-methoxymethyl-1,3-diaminopropan-platin-II-dichlorid (Verbindung 40)
2-Hydroxymethyl-2-para-toluolsulfonyloxymethyl)-1,3-diaminopropan-platin-II-dichlorid (Verbindung 41)
2-2-Bis-(para-toluolsulfonyloxymethyl)-1,3-diaminopropan-platin-II-dichlorid (Verbindung 42)
2,2-Bis-(methansulfonyloxymethyl)-1,3-diaminopropan-platin-II-dichlorid (Verbindung 43)
3,3-Bis-(aminomethyl)-oxetan-platin-II-dichlorid (Verbindung 44)
5,5-Bis-(aminomethyl-1,3-dioxan-platin-II-dichlorid (Verbindung 45)
5,5-Bis-(aminomethyl)-2-phenyl-1,3-dioxan-platin-II-dichlorid (Verbindung 46)
5,5-Bis-(aminomethyl)-2,2-bis-(methyl)-1,3-dioxan-platin-II-dichlorid (Verbindung 47)
5,5-Bis-(aminomethyl)-2-methyl-2-octadecyl-1,3-dioxan-platin-II-dichlorid (Verbindung 48)
5,5-Bis-(aminomethyl)-2-decyl-2-methyl-1,3-dioxan-platin-II-dichlorid (Verbindung 49)

Beispiel 12

α,α′,α″,α‴-Tetraminneopentan-platin-II-dichlorid (Verbindung 50)

0,1 Mol α,α′,α″,α‴-Tetraminneopentan wurde mit 0,2 Mol Kaliumtetrachloroplatinat wie im Beispiel 11 beschrieben, zu Verbindung 50 umgesetzt.

Beispiel 13

5,5-Bis-(aminomethyl)-1,3-dioxan-platin-II-dijodid (Verbindung 51)

2,44 mMol Kaliumtetrachloroplatinat und 2,44 mMol Kaliumjodid wurden in 15 ml Wasser suspendiert. Nach 30 min wurden in die entstandene Lösung unter Rühren 2,44 mMol Verbindung 29, gelöst in 5 ml Wasser, innerhalb von 1 h getropft. Das Reaktionsgemisch wurde 20 h bei Raumtemperatur unter Lichtausschluß gerührt, wobei die Verbindung 51 als Niederschlag ausfiel. Der Niederschlag wurde bei 0 °C abfiltriert, mit Wasser und Ethanol gewaschen und aus Wasser und DMF umkristallisiert. Das Produkt wurde noch 3 Tage im Hochvakuum getrocknet.

Ausbeute : 83 %

Beispiel 14

2,2-Bis-(methoxymethyl)-1,3-diaminopropan-platin-II-hydroxid (Verbindung 52)

a) 24 mMol Verbindung 34 wurden in 500 ml destilliertem, entgastem Wasser suspendiert. Nach Zugabe von 48 mMol Silbernitrat, gelöst in 200 ml Wasser, wurde der Reaktionsansatz 40 h bei Raumtemperatur unter Lichtausschluß gerührt. Dann wurde das ausgefallene Silberchlorid abfiltriert und das Filtrat wurde im Vakkum auf 350 ml Volumen eingeengt. Das resultierende Zwischenprodukt — 2,2-Bis-(hydroxymethyl)-1,3-diaminopropan-platin-II-dinitrat — wurde ohne weitere Reinigungsschritte in die folgende Umsetzung eingesetzt.

b) die Lösung mit dem Dinitrat-Zwischenprodukt wurde unter Rühren mit 25 g Ionenaustausch-Harz

(Dowex, Type 1 × 8 ; Aktivierung mit 10 N NaOH) versetzt. Nach 30 min zeigte das Dünnschichtchromatogramm (n-Butanol/Eisessig/Wasser 5 : 3 : 2 ; Cellulose-Folie), daß der Austausch des Nitrates durch Hydroxyl vollständig abgelaufen war. Nach Abfiltrieren des Harzes wurde das Filtrat zum Trocknen unter Lichtausschluß im Vakuum eingeengt.

Ausbeute : 85 %

Analog wurden folgende Verbindungen hergestellt :

2,2-Bis-(octadecyloxymethyl)-1,3-diamino-platin-II-hydroxid (Verbindung 53)
3,3-Bis-(aminomethyl)-oxetan-platin-II-hydroxid (Verbindung 54)
5,5-Bis-(aminomethyl)-1,3-dioxan-platin-II-hydroxid (Verbindung 55)
5,5-Bis-(aminomethyl)-2,2-bis-(methyl)-1,3-dioxan-platin-II-hydroxid (Verbindung 56)
5,5-Bis-(aminomethyl)-2-methyl-2-octadecyl-1,3-dioxan-platin-II-hydroxid (Verbindung 57)
α, α′, α″, α‴-Tetraminneopentan-platin-II-hydroxid (Verbindung 58)

## Beispiel 15

Ausgehend von Platin-II-hydroxid-Verbindungen 52 bis 58 wurden nach dem folgendem Verfahren Di- und Oligocarbonsäure-Derivate hergestellt.

13 mMol einer Platin-II-Hydroxid-Verbindung wurden in 50 ml destilliertem, entgastem Wasser gelöst. Zu dieser Lösung wurden 13 mMol einer Di- oder Oligocarbonsäure unter Rühren und Lichtausschluß gegeben. Nach 6 Stunden wurden die Reaktionslösung im Vakuum zum Trocknen eingedampft und anschließend im Hochvakuum getrocknet.

Ausbeute : 100 %.

5,5-Bis-(aminomethyl)-2,2-bis-(methyl)-1,3-dioxan-platin-II-anion

| Anion | Verbindung Nr. |
|---|---|
| Malonat | 59 |
| Maleinat | 60 |
| 1,1-Cyclobutan-dicarboxylat | 61 |
| 1,2-Cyclobutan-dicarboxylat | 62 |
| 3-Carboxylphthalat | 63 |
| 4-Carboxylphthalat | 64 |
| 3,6,9-Trioxanona-2,9-dicarboxylat | 65 |
| Polyglutaminsäure | 66 |
| Chondroitinsulfat | 67 |

2,2-Bis-(methoxymethyl)-1,3-diaminopropan-II-anion

| Anion | Verbindung Nr. |
|---|---|
| Malonat | 68 |
| 1,1-Cyclobutan-dicarboxylat | 69 |
| Polyglutaminsäure | 70 |

2,2-Bis-(octadecycloxymethyl)-1,3-diamino-platin-II-anion

| Anion | Verbindung Nr. |
|---|---|
| Malonat | 71 |
| 1,1-Cyclobutan-dicarboxylat | 72 |

3,3-Bis-(aminomethyl)-oxetan-platin-II-anion

| Anion | Verbindung Nr. |
|---|---|
| Malonat | 73 |
| 1,1-Cyclobutan-dicarboxylat | 74 |
| Polyglutaminsäure | 75 |

5,5-Bis-(aminomethyl)-1,3-dioxan-platin-II-anion

| Anion | Verbindung Nr. |
|---|---|
| Malonat | 76 |
| Maleinat | 77 |
| 1,1-Cyclobutan-dicarboxylat | 78 |
| 3-Carboxylphthalat | 79 |
| Polyglutaminsäure | 80 |

5,5-Bis-(aminomethyl)-2-methyl-2-octadecyl-1,3-dioxan-platin-II-anion

| Anion | Verbindung Nr. |
|---|---|
| Malonat | 81 |
| 1,1-Cyclobutan-dicarboxylat | 82 |

α, α′, α″, α‴-Tetraminneopentan-platin-II-anion

| Anion | Verbindung Nr. |
|---|---|
| Malonat | 83 |
| 1,1-Cyclobutan-dicarboxylat | 84 |
| Polyglutaminsäure | 85 |

## Beispiel 16

5,5-Bis-(aminomethyl)-1,3-dioxan-platin-II-diacetat (Verbindung 86)

12 mMol Verbindung 47 werden in 300 ml destilliertem, entgastem Wasser suspendiert. Nach Zugabe von 24 mMol Silberacetat wurde der Reaktionsansatz 40 h bei Raumtemperatur unter Lichtausschluß intensiv gerührt. Dann wurde das ausgefallene Silberchlorid abfiltriert und das Filtrat wurde im Vakuum zum Trockenprodukt eingeengt. Das Produkt wurde noch 3 h im Hochvakuum getrocknet.

Analog wurden, ausgehend von Verbindung 47, die mit Silber-I-trifluoracetat, -trifluorsulfonat, -p-toluolsulfonat oder -perchlorat umgesetzt wurde, folgende Verbindungen hergestellt :

5,5-Bis-(aminomethyl)-1,3-dioxan-platin-II-dianion

| Anion | Verbindung Nr. |
|---|---|
| trifluoracetat | 87 |
| trifluormethansulfonat | 88 |
| p-toluolsulfonat | 89 |
| perchlorat | 90 |

## Beispiel 17

Ausgehend von Verbindung 1 und $CH_3-(OCH_2CH_2)_c-O-SO_2CH_3$ mit c = 3 oder 6 wurden wie im Beispiel 3 beschrieben die Verbindungen 91 und 92 hergestellt. Wie in den Beispielen 10 und 11 beschreiben, wurden die Verbindungen 91 oder 92 in entsprechende Aminoverbindungen überführt und mit Kalkumtetrachloroplatinat komplexiert, wobei die Verbindungen 93 und 94 entstanden.

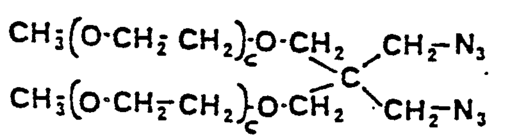

| Verbindung Nr. | c |
|---|---|
| 91 | 3 |
| 92 | 6 |

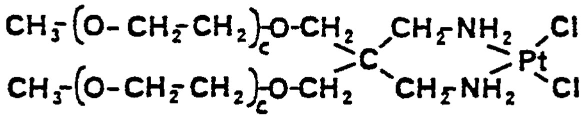

| Verbindung Nr. | c |
|---|---|
| 93 | 3 |
| 94 | 6 |

Tabelle 2 : Elementaranalyse

| Verbindung Nr. | C% | H% | Cl% | N% | Pt% | C% | H% | Cl% | N% | Pt% |
|---|---|---|---|---|---|---|---|---|---|---|
| 34 | 19,63 | 4,24 | 16,56 | 6,54 | 45,56 | 19,51 | 4,19 | 16,12 | 6,51 | 45,13 |
| 35 | 54,41 | 9,58 | 7,83 | 3,09 | 21,55 | 54,14 | 9,49 | 7,84 | 3,01 | 21,64 |
| 36 | 54,41 | 8,00 | 10,42 | 4,12 | 28,66 | 54,40 | 7,96 | 10,31 | 4,03 | 28,34 |
| 37 | 42,33 | 7,72 | 10,86 | 4,29 | 29,89 | 42,18 | 7,64 | 10,69 | 4,16 | 29,17 |
| 38 | 33,34 | 6,34 | 13,12 | 5,18 | 36,10 | 33,16 | 6,33 | 12,96 | 5,16 | 35,97 |

Tabelle 2 (Fortsetzung)

| Verbindung Nr. | C% | H% | Cl% | N% | Pt% | C% | H% | Cl% | N% | Pt% |
|---|---|---|---|---|---|---|---|---|---|---|
| 39 | 43,24 | 7,86 | 10,64 | 4,20 | 29,26 | 43,19 | 7,71 | 10,53 | 4,09 | 29,21 |
| 40 | 34,66 | 6,54 | 12,79 | 5,05 | 35,18 | 34,72 | 6,56 | 12,72 | 5,00 | 34,89 |
| 41 | 26,00 | 3,64 | 12,79 | 5,05 | 35,19 | 26,11 | 3,67 | 12,71 | 5,05 | 35,17 |
| 42 | 32,21 | 3,70 | 10,01 | 3,95 | 27,53 | 32,12 | 3,71 | 10,12 | 3,98 | 27,83 |
| 43 | 15,11 | 3,26 | 12,74 | 5,04 | 35,07 | 15,02 | 3,17 | 12,84 | 4,87 | 35,14 |
| 44 | 15,71 | 3,17 | 18,55 | 7,33 | 51,05 | 15,72 | 3,16 | 18,57 | 7,24 | 51,21 |
| 45 | 17,48 | 3,42 | 17,20 | 6,80 | 47,33 | 17,43 | 3,40 | 17,24 | 6,51 | 46,94 |
| 46 | 30,52 | 3,84 | 15,01 | 5,93 | 41,31 | 30,36 | 3,80 | 15,07 | 5,76 | 41,00 |
| 47 | 21,83 | 4,12 | 16,11 | 6,36 | 44,31 | 21,80 | 4,11 | 16,32 | 6,32 | 44,07 |
| 48 | 46,43 | 8,11 | 10,96 | 4,33 | 30,17 | 46,21 | 8,07 | 10,97 | 4,19 | 30,12 |
| 49 | 36,04 | 6,41 | 12,52 | 4,95 | 34,44 | 36,01 | 6,44 | 12,42 | 4,93 | 34,14 |
| 50 | 9,04 | 2,43 | 21,35 | 8,44 | 58,74 | 9,00 | 2,41 | 21,37 | 8,41 | 58,81 |
| 51 | 12,11 | 2,37 | - | 4,71 | 32,78 | 12,03 | 2,32 | - | 4,63 | 32,16 |
| 52 | 21,41 | 5,15 | - | 7,16 | 49,85 | 21,34 | 5,13 | - | 7 11 | 49,53 |
| 53 | 56,72 | 10,22 | - | 3,23 | 22,47 | 56,72 | 10,19 | - | 3,12 | 22.31 |
| 54 | 17,39 | 4,09 | - | 8,11 | 56,50 | 17,26 | 4,06 | - | 8,02 | 56,41 |
| 55 | 19,20 | 4,30 | - | 7,46 | 51,98 | 19,21 | 4,32 | - | 7,36 | 51,34 |
| 56 | 23,82 | 5,00 | - | 6,95 | 48,37 | 23,73 | 4,93 | - | 6,82 | 48,07 |
| 57 | 46,79 | 8,48 | - | 4,36 | 30,40 | 46,63 | 8,48 | - | 4,32 | 30,29 |
| 58 | 10,17 | 3,41 | - | 9,49 | 66,09 | 10,11 | 3,40 | - | 9,52 | 66,12 |
| 59 | 28,03 | 4,28 | - | 5,94 | 41,39 | 28,01 | 4,27 | - | 5,91 | 41,07 |
| 60 | 29,82 | 4,17 | - | 5,80 | 40,36 | 29,56 | 4,09 | - | 5,78 | 40,19 |
| 61 | 32,88 | 4,73 | - | 5,48 | 38,14 | 32,86 | 4,73 | - | 5,37 | 38,16 |
| 62 | 32,88 | 4,73 | - | 5,48 | 38,14 | 32,88 | 4,71 | - | 5,41 | 38,07 |
| 63 | 35,36 | 3,84 | - | 4,85 | 33,78 | 35,32 | 3,79 | - | 4,80 | 33,69 |
| 64 | 35,36 | 3,84 | - | 4,85 | 33,78 | 35,29 | 3,83 | - | 4,81 | 33,21 |
| 65 | 32,60 | 5,13 | - | 4,75 | 33,09 | 32,60 | 5,12 | - | 4,76 | 33,10 |
| 66 | - | - | - | - | 31,1 | - | - | - | - | 30,27 |
| 67 | - | - | - | - | 14,94 | - | - | - | - | 14,03 |
| 68 | 24,64 | 4,14 | - | 5,75 | 40,03 | 24,61 | 4,16 | - | 5,71 | 39,73 |
| 69 | 31,26 | 4,84 | - | 5,61 | 39,06 | 31,12 | 4,78 | - | 5,49 | 39,01 |
| 70 | - | - | - | - | 31,71 | - | - | - | - | 31,11 |
| 71 | 56,44 | 9,47 | - | 2,99 | 20,84 | 56,42 | 9,46 | - | 2,93 | 20,76 |
| 72 | 57,82 | 9,50 | - | 2,87 | 19,98 | 57,80 | 9,47 | - | 2,90 | 19,43 |
| 73 | 23,25 | 3,41 | - | 6,78 | 47,20 | 23,21 | 3,40 | - | 6,72 | 47,02 |
| 74 | 29,14 | 4,00 | - | 6,18 | 43,03 | 29,07 | 3,99 | - | 6,16 | 43,01 |
| 75 | - | - | - | - | 34,28 | - | - | - | - | 33,85 |
| 76 | 24,38 | 3,64 | - | 6,32 | 44,01 | 24,32 | 3,63 | - | 6,27 | 44,07 |
| 77 | 26,38 | 3,54 | - | 6,15 | 42,15 | 26,38 | 3,54 | - | 6,17 | 42,18 |
| 78 | 29,12 | 4,17 | - | 5,80 | 40,36 | 29,13 | 4,15 | - | 5,81 | 40,43 |

Tabelle 2 (Fortsetzung)

| Verbindung Nr. | C% | H% | Cl% | N% | Pt% | C% | H% | Cl% | N% | Pt% |
|---|---|---|---|---|---|---|---|---|---|---|
| 79 | 32,79 | 3,30 | - | 5,10 | 35,51 | 32,69 | 3,27 | - | 5,03 | 35,36 |
| 80 | - | - | - | - | - | - | - | - | - | - |
| 81 | 47,38 | 7,67 | - | 3,95 | 27,48 | 47,29 | 7,64 | - | 3,82 | 27,17 |
| 82 | 49,65 | 7,80 | - | 3,74 | 26,02 | 49,65 | 7,67 | - | 3,72 | 26,07 |
| 83 | 18,19 | 2,78 | - | 7,71 | 53,71 | 18,19 | 2,69 | - | 7,73 | 53,82 |
| 84 | 25,31 | 3,50 | - | 6,95 | 48,37 | 25,31 | 3,49 | - | 6,94 | 48,12 |
| 85 | - | - | - | - | 37,61 | - | - | - | - | 27,23 |
| 86 | 26,15 | 4,39 | - | 6,10 | 42,47 | 26,15 | 4,38 | - | 6,07 | 42,43 |
| 87 | 17,69 | 2,60 | - | 5,16 | 35,91 | 17,53 | 2,57 | - | 5,11 | 35,90 |
| 88 | 15,03 | 2,21 | - | 4,38 | 30,51 | 15,00 | 2,20 | - | 4,32 | 30,38 |
| 89 | 35,14 | 4,13 | - | 4,10 | 28,54 | 35,17 | 4,11 | - | 4,08 | 28,13 |
| 90 | 13,34 | 2,61 | 13,13 | 5,19 | 36,12 | 13,33 | 2,59 | 13,17 | 5,08 | 36,02 |

**Patentansprüche**

1. Verbindung der allgemeinen Formel I oder II

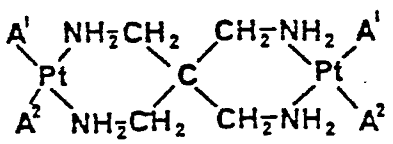

Formel I

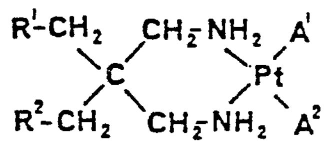

Formel II

worin

$R^1$ und $R^2$ unabhängig voneinander für $H—(CH_2)_a—(0—(CH_2)_{bc}—O$ mit a = 0 bis 4, b = 1 bis 4 und c = 1 bis 7, eine Alkyloxy- oder Arylalkyloxygruppe mit 1 bis 20 C-Atomen, eine Alkansulfonyloxy- oder Arylalkansulfonyloxygruppe mit 1 bis 7 C-Atomen oder einen Tetrahydropyranyloxyrest stehen oder

$R^1$ und $R^2$ zusammen ein etherartig gebundenes Sauerstoffatom oder einen Acetal- oder Ketal-Rest

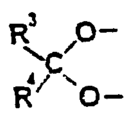

mit

$R^3$ und $R^4$ unabhängig voneinander ein Wasserstoffatom, eine Alkylgruppe mit 1 bis 20 C-Atomen oder eine Phenylgruppe,

$A^1$ und $A^2$ gleich sind und eine Hydroxygruppe, Chlorid, Bromid, Jodid, Nitrat, Acetat, Trifluoracetat, Trifluorsulfonat, oder Perchlorat oder

$A^1$ Sulfat oder Carbonat und $A^2$ $H_2O$ oder

$A^1$ und $A^2$ zusammen das Dianion einer organischen Säure, wie Oxal-, Malon-, Hydroxymalon-, Ethylmalon-, Bernstein-, Malein-, Aconit-, 3,6,9-Trioxaundecandi-, 1,1- oder 1,2-Cyclobutandicarbon-, Phthal-, 3- oder 4-Carboxyphthal- oder 3,4-Dicarboxyphthalsäure oder

$A^1$ und $A^2$ zusammen eine wiederkehrende anionische Einheit einer polymeren Verbindung wie Dextran-, Chondroitin- oder Galactansulfat, Polyglutaminsäure oder Polyitaconsäure darstellen.

2. Verbindung der allgemeinen Formel VI, die als Vorprodukt zur Herstellung der Verbindungen nach Anspruch 1 dient,

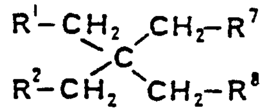

Formel VI

worin $R^1$ und $R^2$ die Bedeutung nach Anspruch 1 haben, $R^7$ und $R^8$ eine Azidogruppe oder eine Aminogruppe, die gegebenenfalls als Ammoniumsalz vorliegt, bedeuten.

3. Verfahren zur Herstellung einer Verbindung nach Anspruch 1, dadurch gekennzeichnet, daß man eine Verbindung der allgemeinen Formel VI, worin

$R^1$ und $R^2$ die unter Anspruch 1 beschriebene Bedeutung besitzen und

$R^7$ und $R^8$ Azidogruppen sind in Gegenwart eines Hydrierungskatalysators wie Palladium/Kohle und eines Lösungsmittels wie Wasser, Methanol, THF, Dioxan, Essigester oder deren Mischungen bei 10 °C bis 40 °C hydriert und das Reaktionsprodukt der Formel VI, in dem $R^1$ und $R^2$ die oben beschriebene Bedeutung beibehalten und $R^7$ und $R^8$ Aminogruppen bzw. deren Ammoniumsalze sind, mit $K_2PtX_4$, worin

X ein Chlorid oder Jodidion darstellt, in Gegenwart eines Lösungsmittels wie Wasser, DMF oder DMSO oder deren Mischungen mit THF, Dioxan, Methanol oder Ethylenglycoldimethylether bei einer Temperatur von 0 °C bis 80 °C in an sich bekannter Weise zu einem Reaktionsprodukt der allgemeinen Formel II umsetzt, in der $R^1$ und $R^2$ die oben angegebene Bedeutung besitzt und $A^1$ uns $A^2$ Chlorid oder Jodid bedeuten, und diese Verbindung gegebenenfalls in eine weitere Verbindung nach Anspruch 1 umsetzt.

4. Verbindung nach Anspruch 1 als Arzneimittel.

**Claims**

1. A compound of the formula I or II

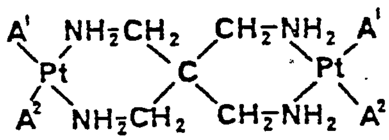

Formula I

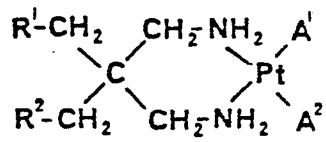

Formula II

in which

$R^1$ and $R^2$ independently of one another represent $H—(CH_2)_a—(0—(CH_2)_b)_c—O$, where $a = 0$ to 4, $b = 1$ to 4 and $c = 1$ to 7, an alkoxy or arylalkoxy group with 1 to 20 carbon atoms, an alkanesulfonyloxy or aralkanesulfonyloxy group with 1 to 7 carbon atoms or a tetrahydropyranyloxy radical, or

$R^1$ and $R^2$ together represent an oxygen atom bonded in an ether-like manner or an acetal or ketal radical

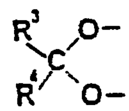

where

$R^3$ and $R^4$ independently of one another represent a hydrogen atom, an alkyl group with 1 to 20 carbon atoms or a phenyl group,

$A^1$ and $A^2$ are identical and represent a hydroxyl group, chloride, bromide, iodide, nitrate, acetate, trifluoroacetate, trifluorosulfonate or perchlorate, or

$A^1$ represents sulfate or carbonate and $A^2$ represents $H_2O$, or

$A^1$ and $A^2$ together represent the dianion of an organic acid, such as oxalic, malonic, hydroxymalonic, ethylmalonic, succinic, maleic, aconitic, 3,6,9-trioxaundecanedioic, 1,1- or 1,2-cyclobutanedicarboxylic, phthalic, 3- or 4-carboxyphthalic or 3,4-dicarboxyphthalic acid, or

$A^1$ and $A^2$ together represent a recurring anionic unit of a polymeric compound, such as dextran sulfate, chondroitin sulfate or galactan sulfate, polyglutamic acid or polyitaconic acid.

2. A compound of the formula VI used as an intermediate for the preparation of a compound as claimed in claim 1

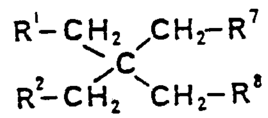

Formula VI

in which $R^1$ and $R^2$ have the meaning as claimed in claim 1 and $R^7$ and $R^8$ denote an azido group or an amino group, which is optionally in the form of an ammonium salt.

3. A process for the preparation of a compound as claimed in claim 1, which comprises hydrogenating a compound of the formula VI, in which

$R^1$ and $R^2$ have the meaning as claimed in claim 1 and

$R^7$ and $R^8$ are azido groups, in the presence of a hydrogenation catalyst, such as palladium-on-charcoal, and a solvent, such as water, methanol, tetrahydrofuran, dioxane, ethyl acetate or a mixture thereof, at 10 °C to 40 °C, reacting the reaction product of the formula VI, in which $R^1$ and $R^2$ contain the meaning described above and $R^7$ and $R^8$ are amino groups or ammonium salts thereof, with $K_2PtX_4$, in which

X represents a chloride or iodide ion, in the presence of a solvent, such as water, dimethylformamide or dimethylsulfoxide or a mixture thereof with tetrahydrofuran, dioxane, methanol or ethylene glycol dimethyl ether, at a temperature of 0 °C to 80 °C in a manner which is known per se to give a reaction product of the formula II, in which $R^1$ and $R^2$ have the abovementioned meaning and $A^1$ and $A^2$ denote chloride or iodide, and, if appropriate, converting this compound into another compound as claimed in claim 1.

4. A compound as claimed in claim 1 as a medicament.

**Revendications**

1. Composé de formule générale I ou II,

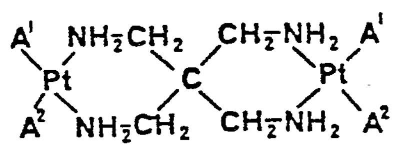

Formule I

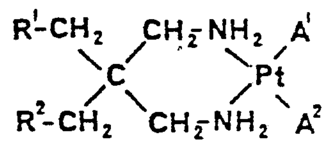

Formule II

dans lesquelles

$R^1$ et $R^2$ représentent, indépendamment l'un de l'autre, $H—(CH_2)_a—(O—(CH_2)_b)_c—O$, a valant de 0 à 4, b valant de 1 à 4 et c valant de 1 à 7, un groupe alkyloxy ou arylalkyloxy ayant de 1 à 20 atomes de carbone, un groupe alcanesulfonyloxy ou arylalcanesulfonyloxy ayant de 1 à 7 atomes de carbone, ou un radical tétrahydropyrannyloxy, ou

$R^1$ et $R^2$ représentent ensemble un atome d'oxygène lié en liaison éther ou un radical acétal ou cétal

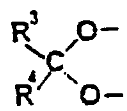

$R^3$ et $R^4$ étant, indépendamment l'un de l'autre, un atome d'hydrogène, un groupe alkyle ayant de 1 à 20 atomes de carbone ou un groupe phényle,

$A^1$ et $A^2$ sont identiques et représentent un groupe hydroxy ou un ion chlorure, bromure, iodure, nitrate, acétate, trifluoroacétate, trifluorosulfonate ou perchlorate, ou

$A^1$ représente l'ion sulfate ou carbonate et $A^2$ représente $H_2O$, ou

$A^1$ et $A^2$ représentent ensemble le dianion d'un acide organique, tel que l'acide oxalique, malonique, hydroxymalonique, éthylmalonique, succinique, maléique, aconitique, 3,6,9-trioxa-undécanedioïque, 1,1- ou 1,2-cyclobutanedicarboxylique, phtalique, 3- ou 4-carboxyphtalique ou 3,4-dicarboxyphtalique, ou

$A^1$ et $A^2$ représentent ensemble un motif anionique répétitif d'un composé polymère, tel que le dextrane-, le chondroïtine- ou le galactane-sulfate, l'acide polyglutamique ou l'acide polyitaconique.

2. Composé de formule générale VI, utilisé en tant que précurseur dans la préparation des composés selon la revendication 1,

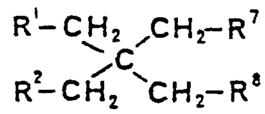

Formule VI

dans laquelle $R^1$ et $R^2$ ont les significations données dans la revendication 1, $R^7$ et $R^8$ représentent un groupe azido ou groupe amino, qui se trouve éventuellement sous forme de sel d'ammonium.

3. Procédé pour la préparation d'un composé selon la revendication 1, caractérisé en ce que l'on soumet un composé de formule générale VI, dans lequel

$R^1$ et $R^2$ ont les significations données dans la revendication 1, et

$R^7$ et $R^8$ sont des groupes azido, à une hydrogénation, à 10-40 °C, en présence d'un catalyseur d'hydrogénation tel que le charbon palladié, et d'un solvant tel que l'eau, le méthanol, le THF, le dioxanne, l'acétate d'éthyle ou des mélanges de ceux-ci, et on fait réagir le produit de réaction de formule VI, dans lequel $R^1$ et $R^2$ ont les significations données plus haut, et $R^7$ et $R^8$ sont des groupes amino ou leurs sels d'ammonium, avec $K_2PtX_4$,

X représentant l'ion chlorure ou iodure, en présence d'un solvant, tel que l'eau, le DMF ou le DMSO, ou de mélanges de ceux-ci avec le THF, le dioxanne, le méthanol ou l'éther diméthylique d'éthylèneglycol, pour aboutir à un produit de réaction de formule générale II, dans lequel $R^1$ et $R^2$ ont les significations données plus haut et $A^1$ et $A^2$ représentent l'ion chlorure ou iodure, et éventuellement on convertit ce composé en un autre composé selon la revendication 1.

4. Composé selon la revendication 1, en tant que médicament.